# EUROPEAN PATENT APPLICATION

(11) **EP 2 783 681 A1**
(43) Date of publication of application: **01.10.2014**
(21) Application number: 14161320.8
(22) Date of filing: 24.03.2014
(51) Int. Cl.: A61K 9/20

(54) **Extended release formulations of metformin**

(30) Priority: 25.03.2013 TR 201303559; 15.07.2013 TR 201308527
(71) Applicant: Sanovel Ilac Sanayi ve Ticaret A.S., 34460 Istanbul (TR)
(72) Inventor: Türkyilmaz, Ali, 34460 Istanbul (TR); Önder, Ramazan, 34460 Istanbul (TR); Eceoglu, Melike, 34460 Istanbul (TR)
(74) Representative: Sevinç, Erkan

(57) **Abstract**

The present invention relates to extended release formulations comprising metformin or a pharmaceutically acceptable salt thereof. The present invention more particularly relates to an extended release formulation of metformin providing the ratio of high viscosity hydroxypropyl methylcellulose to low viscosity hydroxypropyl methylcellulose in the range of 5 - 20 (w/w).

## Description

### Field of Invention

The present invention relates to extended release formulations comprising metformin or a pharmaceutically acceptable salt thereof. The present invention more particularly relates to an extended release formulation of metformin providing the ratio of high viscosity hydroxypropyl methylcellulose to low viscosity hydroxypropyl methylcellulose in the range of 5 - 20 (w/w).

### Background of Invention

Metformin hydrochloride, with the chemical name 1,1-dimethylbiguanide hydrochloride, has the following chemical structure of Formula I.

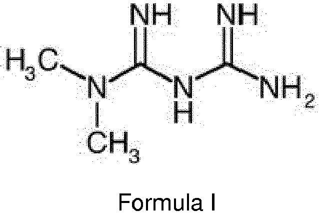

Metformin is an oral antidiabetics having an orally-administrated biguanide structure. It is used singly or in combination with sulfonylureas, alpha-glucosidase inhibitors, or insulin.

Various patent applications are available in relation to metformin; metformin is disclosed with the patent US 3 174 901 and its prolonged release pharmaceutical formulation is disclosed with US 6 475 521.

There is metformin XR formulation licensed in the US by Bristol Myers and it is currently commercially available in strengths of 500 and 750 mg of metformin HCl under the brand name Glucophage® XR. However, there is still a need for metformin XR formulation at higher dosages.

Oral bioavailability of metformin is in the range of 40 to 60 % decreasing with increasing dose and it is highly soluble in water. Having high water solubility leads fast dissolution (rapid release) of the drug. To adjust the dissolution profile, HPMC has been used as a retardant agent in prior art and in the case of dissolution is too fast, the amount of HPMC may be increased, total viscosity may be increased or/and tablet size may be increased to achieve slow dissolution.

High amount of HPMC is required to form an effective matrix, to prevent fast dissolution of a drug. However, using high amount of HPMC cause undesirable size of dosage forms and decrease in bioavailability. Therefore, it is very crucial to choose the exact amount of excipients and ratio of them to each other.

On the other side, viscosity of HPMC affects gel strength (or erosion rate) and hydration speed of the gel and also compressibility of a tablet. The higher the viscosity, the stronger the gel strength and the slower the hydration speed. However, granulation and compression with high viscosity HPMC is difficult. Accordingly, it is difficult to choose HPMC with desired viscosity.

Extended release formulation is not always easy to achieve also in terms of particle size, aggregation and large particle size can be occurred during the process. Metformin with finer particle size is needed to achieve desired dissolution. Finer particle size also gives homogeneity and high flowability properties to the formulation during the process. However, it has been also known that extremely small sizes (less than 10 µm) may be inadvisable for some drug substances.

In accordance with these, there is a need in the prior art for extended release formulation of metformin or pharmaceutically acceptable salts and all said parameters should be optimum in order to overcome fast dissolution and process problems.

### Description of the invention

The main embodiment of the present invention is to provide extended release formulations comprising metformin or a pharmaceutically acceptable salt thereof and pharmaceutically acceptable excipients characterized in that, said formulation comprising high viscosity hydroxypropyl methylcellulose and low viscosity hydroxypropyl methylcellulose and the ratio of high viscosity hydroxypropyl methylcellulose to low viscosity hydroxypropyl methylcellulose is in the range of 5 - 20 (w/w).

Generally, for tablet formulations, high amount of HPMC is not preferred because it leads large size of tablet dosage and low bioavailability which are not desired for patient compliance. For metformin XR formulation, although an increase in HPMC is expected to reach a higher dosage extended release tablet than in the state of art, in this invention, surprisingly without increasing the amount of HPMC, extended release tablet has been achieved at the high dosage (1000 mg or more).

According to the main embodiment, the present invention relates to extended release formulations of metformin providing the ratio of high viscosity hydroxypropyl methylcellulose to low viscosity hydroxypropyl methylcellulose in the range of 5 - 20 (w/w), preferably 10 - 15 (w/w).

As used herein, the term "viscosity" means that the property of resistance to flow in a fluid or resistance of a fluid to a change in shape, or movement of neighboring portions relative to one another. "High viscosity" is defined as strong resistance to flowing and "low viscosity" is defined as high flowability. While high viscosity gives HPMC properties such as strong gel strength and slow release of the drug, HPMC with low viscosity provides easy granulation and compression. Therefore, optimization of viscosity grade provides the desired dissolution and compressibility properties. In this invention, both high and low viscosity HPMC was used to have extended release formulation of metformin. Firstly, granulation with low viscosity HPMC was achieved to increase the compressibility and the homogeneity of metformin, then high viscosity HPMC was used to obtain slow release. By changing the viscosity grade and proportions of HPMCs with different viscosity, desired viscosity and dissolution has been achieved while compressibility has been enhanced surprisingly.

In one embodiment, high viscosity HPMC is K100 MCR that has 75.000-140.000 cps viscosity grades with the ratio of 1 - 50 %, preferably 10 - 30 % by weight of total formulation. Low viscosity HPMC is K100 LV that has 80-120 cps viscosity grades.

According to one embodiment, finer particle size of metformin shows slow dissolution profile and has advantages over flowability and homogeneity. On the other hand, very fine particle size is not desired during the press of granules or powders into tablet form and finer size may limit the dissolution rate or drug surface-liquid contact. In this invention, micronized metformin which has the median particle size between 20 to 45 µm, preferably 20 to 35 and more preferably 20 to 25 µm was desired.

"Median particle size" is defined by "d₅₀", the size at which 50% by volume of the particles are finer. In this invention, d₅₀ value of metformin has been measured by *Malvern Mastersizer 2000* laser diffraction particle size analyzer.

According to one embodiment of the present invention is to provide the extended release formulations wherein metformin or a pharmaceutically acceptable salt is present in an amount of about 50 to 98 % by weight of total formulation, preferably it is about 60 to 85 % by weight of total formulation.

According to another embodiment of this invention, extended release formulation comprises at least one pharmaceutically acceptable excipient is selected from the group comprising diluents, disintegrants, binders, lubricants, glidants or the mixtures thereof.

Suitable diluents are selected from the group comprising dibasic calcium phosphate, tribasic calcium phosphate, trehalose, isomalt, microcrystalline cellulose, mannitol, lactose, starch, sodium carbonate, sodium bicarbonate, calcium carbonate or mixtures thereof.

Suitable disintegrants are selected from the group comprising croscarmellose sodium, sodium carboxymethyl starch, sodium starch glycolate, soy polysaccharide, cross-linked alginic acid, gellan gum, xanthan gum, calcium silicate or ion exchange resins or mixtures thereof.

The binders are selected from the group comprising at least one of sodium carboxymethyl cellulose, polyvinylpyrrolidone, hydroxypropyl methylcellulose, hydroxypropyl cellulose, sodium carboxymethyl cellulose, methyl cellulose, ethyl cellulose or other cellulose derivatives, or gelatin or mixtures thereof, preferably sodium carboxymethyl cellulose.

In a further embodiement, sodium carboxymethyl cellulose is used as a binder to further increase the compressibility and modify the viscosity. The amount of sodium carboxymethyl cellulose is in the range of 1 - 10%, preferably 3 to 5 % by weight of total formulation and the ratio of metformin to sodium carboxymethyl cellulose is in the range of 5 - 100 (w/w), preferably 5 - 50 (w/w), more preferably 15 - 25 (w/w).

Suitable lubricants are selected from the group comprising magnesium stearate, sodium stearyl fumarate, polyethylene glycol, leucine, poloxamer, sodium benzoate or sodium lauryl sulfate or mixtures thereof, preferably magnesium stearate.

According to one embodiment, the amount of magnesium stearate is in the range of 0.2 - 10%, preferably 0.4 to 5 % by weight of total formulation.

Suitable glidants are selected from the group comprising colloidal silicon dioxide, talc, aluminium, silicate or mixtures thereof.

In this present invention, to improve tablet compressibility and achieve desired dissolution profile, an extended release formulation has been designed, comprising following:
a) 60 to 85% by weight of metformin
b) 3 to 5 % by weight of sodium carboxymethyl cellulose
c) 1 to 3 % by weight of hydroxypropyl methylcellulose (K100 LV)
d) 10 to 30 % by weight of hydroxypropyl methylcellulose (K100 MCR)
e) 0.4 to 5 % by weight of magnesium stearate

### Example 1: Metformin XR tablet

| **Ingredients** | **% amount** |
|---|---|
| Metformin | 74.0 % |
| sodium carboxymethyl cellulose | 3.7 % |
| hydroxypropyl methylcellulose (K100 LV) | 1.5 % |
| hydroxypropyl methylcellulose (K100 MCR) | 20.3 % |
| Magnesium stearate | 0.5 % |

The production of the formulation is carried out by wet granulation as follows: Metformin added to the Collette and granules were crushed for 5 minutes. Sodium carboxymethyl cellulose and hydroxypropyl methylcellulose (K100 LV) were added to granulator and mixed for 10 minutes. Wet granulation is obtained by water. Granules are sieved and dried in fluid bed dryer. Then, dried granules are sieved and mixed with hydroxypropyl methylcellulose (K100 MCR) for 15 minutes. Then, magnesium stearate was added to mixer and mixed for 3 minutes. Total mixture was pressed into tablets.

With this invention, an extended release formulation comprising metformin or a pharmaceutically acceptable salt thereof has been achieved eliminating any dissolution and process related problems without decrease in bioavailability.

## Claims

1. An extended release formulation comprising therapeutically effective amount of metformin or a pharmaceutically acceptable salt thereof and pharmaceutically acceptable excipients **characterized in that**,
a) said formulation comprising high viscosity hydroxypropyl methylcellulose and low viscosity hydroxypropyl methylcellulose,
b) the ratio of high viscosity hydroxypropyl methylcellulose to low viscosity hydroxypropyl methylcellulose is in the range of 5 - 20 (w/w).

2. The extended release formulation according to claim 1, wherein the viscosity of high viscosity hydroxypropyl methylcellulose has between 75.000-140.000 cps viscosity grades with the ratio of 1 - 50 %.

3. The extended release formulation according to claim 1, wherein the pharmaceutically acceptable excipients are selected from the group comprising diluents, disintegrants, binders, lubricants, glidants or the mixtures thereof.

4. The extended release formulation according to claim 4, wherein said binders are selected from the group comprising sodium carboxymethyl cellulose polyvinylpyrrolidone, hydroxypropyl methylcellulose, hydroxypropyl cellulose, methyl cellulose, ethyl cellulose or gelatin or mixtures thereof, preferably sodium carboxymethyl cellulose.

5. The extended release formulation according to claim 5, wherein the amount of sodium carboxymethyl cellulose is in the range of 1 - 10%, preferably 3 to 5 % by weight of total formulation

6. The extended release formulation according to any of the preceding claims, wherein the ratio of metformin to sodium carboxymethyl cellulose is in the range of 5 - 100 (w/w), preferably 5 - 50 (w/w), more preferably 15 - 25 (w/w).

7. The extended release formulation according to any of the preceding claims, wherein said lubricants are selected from the group comprising magnesium stearate, sodium stearyl fumarate, polyethylene glycol, leucine, poloxamer, sodium benzoate or sodium lauryl sulfate or mixtures thereof, preferably magnesium stearate

8. The extended release formulation according to claim 8, wherein the amount of magnesium stearate is in the range of 0.2 - 10%, preferably 0.4 to 5% by weight of total formulation.

9. The extended release formulation according to any preceding claims comprising ;
a) 60 to 85% by weight of metformin
b) 3 to 5 % by weight of sodium carboxymethyl cellulose
c) 1 to 3 % by weight of hydroxypropyl methylcellulose (low viscosity)
d) 10 to 30 % by weight of hydroxypropyl methylcellulose (high viscosity)
e) 0.4 to 5 % by weight of magnesium stearate

10. The extended release formulation according to any preceding claims comprising ;
a) 74% by weight of metformin
b) 3.7 % by weight of sodium carboxymethyl cellulose
c) 1.5 % by weight of hydroxypropyl methylcellulose (low viscosity)
d) 20.3 % by weight of hydroxypropyl methylcellulose (high viscosity)
e) 0.5 % by weight of magnesium stearat

11. Method for preparing the extended release formulation according to any of the preceding claims, comprising the steps of;
a) metformin added to the Collette and granules were crushed for 5 minutes.
b) sodium carboxymethyl cellulose and hydroxypropyl methylcellulose (low viscosity) were added to granulator and mixed for 10 minutes.
c) wet granulation is obtained by water.
d) granules are sieved and dried in fluid bed dryer.
e) dried granules are sieved and mixed with hydroxypropyl methylcellulose (high viscosity) for 15 minutes.
f) magnesium stearate was added to mixer and mixed for 3 minutes.

12. The extended release formulation according to any of the preceding claims, wherein metformin has the median particle size between 20 to 45 µm, preferably 20 to 35 and more preferably 20 to 25 µm.
